# EUROPEAN PATENT SPECIFICATION

(11) **EP 0 698 384 B1**
(45) Date of publication and mention of the grant of the patent: **21.03.2001**
(21) Application number: 94202428.2
(22) Date of filing: 24.08.1994
(51) Int. Cl.: A61F 13/15

(54) **Disposable absorbent article with self adapting body facing surface topography**
Absorbierender Wegwerfartikel mit selbstadaptierender Körperflächenseite
Article absorbant jetable auto-adaptable à la forme du corps

(43) Date of publication of application: 28.02.1996
(73) Proprietor: THE PROCTER & GAMBLE COMPANY, Cincinnati, Ohio 45202 (US)
(72) Inventor: Plumley, Julian Ashton, D-74589 Satteldorf (DE)
(74) Representative: Hirsch, Uwe Thomas

(56) References cited:
- EP-A- 0 359 391
- DE-A- 3 517 192
- GB-A- 1 575 363

## Description

### Field of the invention:

The present invention relates to disposable absorbent articles comprising a gas bubble to raise the body facing surface of the article towards the wearer especially in the area of liquid discharge. In particular the present invention relates to sanitary napkins, catamenials incontinence inserts and pantyliners comprising an air bubble between backsheet and absorbent core to raise the absorbent core and the topsheet towards the perineal area of female users. The gas bubble is filled with gas such that it yields to external pressures in order to maintain a comfortable fit of the absorbent article.

### Background of the invention

The basic problem of achieving an absorbent article which closely contacts the body surface around the liquid discharge area of wearers of such articles has been addressed by various designs heretoforth. However, the designs and features suggested in this respect can be further improved for comfort and convenience in respect to the widely varying surface topography of different wearers. This is underlined by the lack of widely available products having features which raise the body facing surface towards the wearer.

WO-9207535 discloses topsheet raising devices in the form of resilient longitudinal spacing structures. The spacing structure is activated by side pressure from the thighs of the wearer. WO-9014063 discloses a hose-like absorption body which also spaces the topsheet away from the absorbent core thereby producing a void space for additional absorption capacity in an absorbent product. US-4,846, 824 discloses a labia-pad which is anatomically conformed by folding and confining the product into a raised shape. None of these three prior art disclosures refers to hermetically enclosed gas bubbles to provide the desired shaping function.

GB-1,462,467 discloses an absorbent pad construction designed for heavy-load bearing bed pads. GB-1,575, 363 discloses bed pads or sanitary dressings having a backsheet of particular resiliency. US-4,723,953 also discloses a particularly soft bed pad construction showing good resiliency. DE-3517192 discloses a napkin for feminine hygiéne and incontinence which is elastically stabilized in a given form by a sealed system of air cushion canal. These documents all refer to a bubble pack type of construction of the impermeable backsheet in order to provide resiliency for the whole of the Absorbent article. No particular area in the topsheet is raised to register with any part of the human body according to these disclosures.
US-3,881, 491 and US-3,921,232 both relate to activatable gas bubbles designed to provide resiliency and form the absorbent article surface into such a shape that it provides for particular benefits in acquiring bodily exudates. Both documents particularly relate to the separation of feces by providing pockets within the body-facing surface of the absorbent article. These designs have the drawback that they need an activation liquid ,usually urine. On top of that, the uncertainty in gas amount created raises questions of consistency of design and function.

It is therefore an objective of the present invention to provide an absorbent article with a gas bubble which is yielding so as to comfortably conform the body-facing surface of the article to the individual topography of the wearer in the area of liquid discharge.

### Brief description of the invention

According to the present invention a disposable article for absorbing liquid by placing it adjacent a body discharge area is provided. The article has a body-facing surface, typically provided by a liquid permeable substrate of fibrous or film-like structure; a garment facing surface, typically provided by a liquid impermeable substrate and an absorbent core placed between the body facing surface and the garment facing surface. The absorbent article has a longitudinal and a lateral axis and comprises a yielding gas bubble or a number of gas bubbles which function in combination to raise the body facing surface towards the liquid discharge area of the wearer according to the characterising features of claim 1. The combined shape and size of the bubble(s) may vary considerably from round to rectangular and elliptical shapes or even combinations thereof.

The gas bubble is preferably placed between the absorbent core and the garment facing surface in order to raise the body facing surface without obstructing the liquid passage to the core while raising the core to also follow the body facing surface topography. The gas bubble is preferably contained in an essentially gas impermeable material like polyethylene, polypropylene, polyethylen terephtalat, aluminium or mixtures or laminates of these materials.

### Description of the drawing

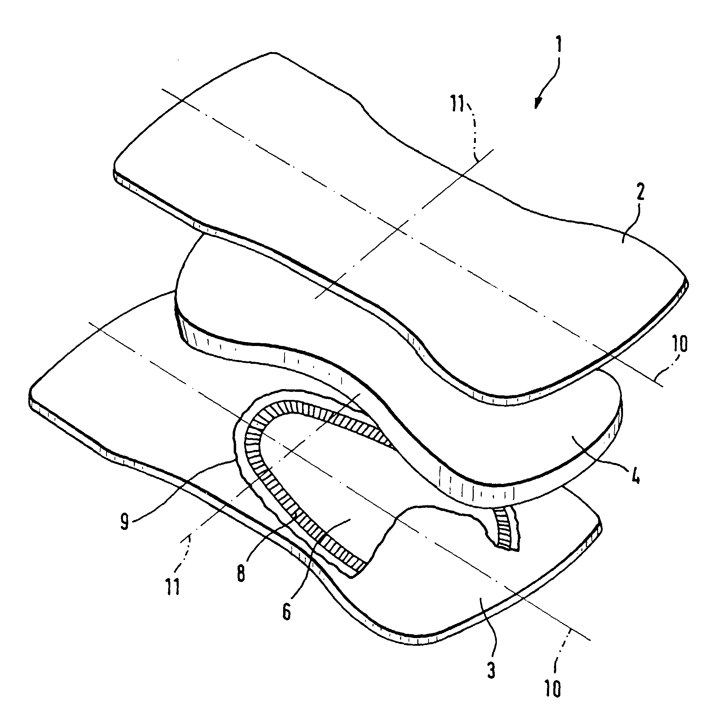

Figure 1 shows an exploded view of a preferred embodiment of a sanitary napkin according to the present invention comprising an air bubble integrally formed on the backsheet below the absorbent core.

### Detailed description of the invention

The disposable article for absorbent liquid is described below by reference to a sanitary napkin or catamenial. However products such as baby diapers, adult incontinence inserts, or pantyliners may similarly be supplied with the characterising feature of the present invention. A preferred embodiment of a sanitary napkin according to the present invention is shown in a exploded view in Figure 1. A liquid permeable topsheet (2) overlays an absorbent core (4) which overlays a liquid impermeable backsheet (3). An air bubble (6) is formed by joining the material forming the skin of the air bubble along an endless line (8) to the backsheet (3). The endless line (8) is close to the perimeter (9) of the skin forming material of the air bubble (6) but not necessarily coinciding with the perimeter (9).

### Gas bubble(s)

The gas bubble(s) can comprise essentially any gas desired but preferably air. he bubble can be formed from any material which provides a sustainable internal amount of air over the expected product lifetime from manufacturing of the absorbent product to the disposal thereof.

The material used for the bubble should be soft and compliant in order to allow the deformation of the bubble. Typical materials can be those also used for impervious backsheets as well as polyethylene terephtalat and aluminium or mixtures and laminates of these materials.

The bubble may be formed separately or as an integral part of the backsheet as shown in Figure 1. If formed separate from the backsheet it is desired to immobilise the bubble in relation to the product such that it cannot dislocate during manufacturing, transport, storage and use of the absorbent product. This may be achieved in any usual way but typically would by means of an adhesive. If the gas bubble is formed as an integral part of the backsheet the backsheet must also be impermeable to the gas with which the bubble is filled.

The bubble may be placed between the topsheet (2) and the absorbent core (4) but could then obstruct the liquid passage from the liquid permeable topsheet to the absorbent core. Therefore it preferably is placed between the absorbent core (4) and the backsheet (3).

The gas bubble is typically longer along the longitudinal axis (10) than wide along the lateral axis (11). Also the bubble may have a variety of shapes which are typically symmetrical to the longitudinal axis (10) but asymmetrical to the lateral axis (11). The function of the bubble may also be provided by a number of smaller individual bubbles which in combination provide the desired shape. In particular 2 or 3 bubbles to allow a gradient of the amount of raising the body facing surface and folding lines between the bubbles can replace a single bubble.

If the gas bubble is formed integral with the backsheet as shown in Figure 1 the joining along line 8 needs to be gas impermeable. This joining may be achieved by such methods as welding, ultrasonic bonding or adhesive attachment.

The bubble is typically filled such that the filling volume is substantially below its maximum filling volume in order to provide a wobbly, flexible and easily deformable outer surface. This ability to deform, also referred to as yielding, provides the desired exceptional ability to adapt the surface topography of the bubble and the overlying absorbent core and topsheet to the topography of the individual wearer of an absorbent product according to the present invention. By filling the bubble no more than 90 %, preferably no more than 80 % of the maximum volume of the bubble it is possible to ensure that even under the temperature changes common during the wearing of such a product the air bubble will continue to be yielding such that it adapts the product to the wearer's topography.

### Topsheet

The topsheet (2) is compliant, soft feeling, and non-irritating to the wearer's skin. The topsheet also can have elastic characteristics allowing it to be stretched in one or two directions. Further, the topsheet is fluid pervious permitting fluids (e.g., menses and/or urine) to readily penetrate through its thickness. A suitable topsheet can be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers.

Preferred topsheets for use in the present invention are selected from high loft nonwoven topsheets and aperture formed film topsheets. Apertured formed films are especially preferred for the topsheet because they are pervious to body exudates and yet non-absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in US-3,929,135; US-4,324,246; US-4,342,314; US-4,463,045; and US-5,006,394. Particularly preferred microapetured formed film topsheets are disclosed in US-4,609,518 and US-4,629,643. The preferred topsheet for the present invention is the formed film described in one or more of the above patents and marketed on sanitary napkins by The Procter & Gamble Company of Cincinnati, Ohio as "DRI-WEAVE."

Topsheets having not a homogeneous distribution of liquid passage ways but only a portion of the topsheet comprising liquid passage ways are also contemplated by the present invention.

Typically such topsheets would have the liquid passage ways oriented such that they result in a centrally permeable and peripherally impermeable topsheet for liquids.

The body surface of the formed film topsheet can be hydrophilic so as to help liquid to transfer through the topsheet faster than if the body surface was not hydrophilic. In a preferred embodiment, surfactant is incorporated into the polymeric materials of the formed film topsheet such as is described in WO-93 09741. Alternatively, the body surface of the topsheet can be made hydrophilic by treating it with a surfactant such as is described in US-4,950,254.

### Absorbent core

The absorbent core is shown as a single entity (4) in Figure 1. It can include the following components: (a) optionally a primary fluid distribution layer preferably together with a secondary fluid distribution layer; (b) a fluid storage layer; (c) optionally a fibrous ("dusting") layer underlying the storage layer; and (d) other optional components.

The gas bubble typically is placed between the core and the backsheet or the topsheet. However for cores constructed of more than one layer the gas bubble can also be placed between the layers of the core. Also the gas bubble may reach into the core by having the respective space provided in a particular layer. Generally, but in particular if layered core structures of the laminate type are employed, the core should allow the bubble to provide it's yielding function. This can be ensured by rendering the core extensible at least in the region adjacent the gas bubble.

### a. Primary / Secondary Fluid Distribution Layer

One optional component of the absorbent cores according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized.

### b. Fluid Storage Layer

Positioned in fluid communication with , and typically underlying the primary or secondary distribution layers, is a fluid storage layer. The fluid storage layer can comprise any usual absorbent material or combinations thereof. It preferable comprising absorbent gelling materials usually referred to as "hydrogels," "superabsorbent" "hydrocolloid" materials.

These absorbent gelling materials are capable of absorbing large quantities of aqueous body fluids, and are further capable of retaining such absorbed fluids under moderate pressures. These absorbent gelling materials can be in the form of discrete particles. The absorbent gelling materials can be dispersed homogeneously or non-homogeneously in a suitable carrier.

Suitable absorbent gelling materials for use herein will most often comprise a substantially water-insoluble, slightly crosslinked, partially neutralized, polymeric gelling material. This material forms a hydrogel upon contact with water. Such polymer materials can be prepared from polymerizable, unsaturated, acid-containing monomers. Suitable unsaturated acidic monomers for use in preparing the polymeric absorbent gelling material used in this invention include those listed in US-4,654,039 reissued as RE 32,649. Preferred monomers include acrylic acid, methacrylic acid, and 2-acrylamido-2-methyl propane sulfonic acid. Acrylic acid itself is especially preferred for preparation of the polymeric gelling material.

Suitable carriers include materials which are conventionally utilized in absorbent cores such as cellulose fibers, in the form of fluff. Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., that lower rewet problems.

If dispersed non-homogeneously in a carrier, the storage layer can be locally homogeneous, i.e. have a distribution gradient in one or several directions within the dimensions of the storage layer. Non-homogeneous distribution can also refer to laminates of carriers enclosing absorbent gelling materials partially or fully.

### c. Optional Fibrous ("Dusting") Layer

An optional component for inclusion in the absorbent cores according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macrostructures such as fibers, sheets or strips, this fibrous "dusting" layer need not be included. However, this "dusting" layer provides some additional fluid-handling capabilities such as rapid wicking of fluid along the length of the pad.

### d. Other Optional Components

The absorbent cores according to the present invention can include other optional components normally present in absorbent webs. For example, a reinforcing scrim can be positioned within the respective layers, or between the respective layers, of the absorbent cores. Such reinforcing scrims should be of such configuration as to not form interfacial barriers to fluid transfer, especially if positioned between the respective layers of the absorbent core. Given the structural integrity that usually occurs as a result of thermal bonding, reinforcing scrims are usually not required for the absorbent structures according to the present invention.

Another component which can be included in the absorbent core according to the invention and preferably is provided close to or as part of the primary or secondary fluid distribution layer are odor control agents. Typically active carbon coated with or in addition to other odor control agents, in particular suitable zeolite or clay materials, are optionally incorporated in the absorbent core. These components can be incorporated in any desired form but often are included as discrete particles.

### Backsheet

The backsheet (3) prevents the exudates absorbed and contained in the absorbent core from wetting articles that contact the absorbent product such as underpants, pants, pajamas and undergarments. The backsheet (3) is preferably impervious to liquids (e.g., menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet can comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance. Further, the backsheet can permit vapors to escape from the absorbent structure (i.e., be breathable) while still preventing exudates from passing through the backsheet.

If the backsheet forms part of the skin of the gas bubble according to the present invention it needs to be impermeable at least in the area of the gas bubble.

### Optional components of the absorbent products

Optionally, the absorbent product of the present invention can comprise all those components typical for the particularly intended product use. For example catamenials, panty liners and sanitary napkins often comprise components such as wings and panty fastening adhesives in order to improve their positioning and soiling protection performance. Leg elastication by one or several elastic strands is also common in the art of absorbent products. In general, all typically used components in absorbent products can also be comprised in the absorbent products according to the present invention as long as a gas bubble is present.

## Claims

1. Disposable absorbent article for wearing adjacent a body discharge area, said article having a body facing surface , a garment facing surface and an absorbent core between said body facing surface and said garment facing surface and further having a longitudinal axis and a lateral axis, said article comprising a yielding gas bubble or a number of yielding gas bubbles which function in combination, said article being characterized in that the gas bubble(s) is (are) filled on manufacture exclusively with gas up to no more than 90 % of the maximum volume of the (each) gas bubble and has (have) a (combined) shape which has an orientation which is symmetrical to the longitudinal axis but asymmetrical to the lateral axis of the article so as to raise said body facing surface towards the discharge area for adaptation of said body facing surface to the topography of the wearer in that area.

2. Disposable absorbent article according to claim 1 characterized in that said gas is air.

3. Disposable absorbent article according to any of the preceding claims characterized in that said gas bubble(s) is (are) enclosed in a film, the material of said film preferably being selected from the group of polyethylene, polypropylene, polyethyleneterephthalat, aluminium, mixtures thereof or laminates thereof.

4. Disposable absorbent article according to any of the preceding claims characterized in that said gas bubble or that the combined shape of the gas bubbles is longer along said longitudinal axis than along said lateral axis.

5. Disposable absorbent article according to any of the preceding claims characterized in that said (combined) gas bubble(s) has (have) a shape selected from the group of rectangular shapes, round shapes, elliptical shapes or combinations thereof.

6. Disposable absorbent article according to any of the preceding claims characterized in that said body facing surface is provided by a liquid permeable substrate and said garment facing surface is provided by a liquid impermeable substrate.

7. Disposable absorbent article according to any of the preceding claims characterised in that said gas bubble(s) is (are) placed between said garment facing surface and said absorbent core.

8. Disposable absorbent article according to claim 7 characterized in that said garment facing surface is provided by a liquid and gas impermeable film which film also forms part of said gas bubble(s).

9. Disposable absorbent article according to any of the preceding claims characterized in that said disposable absorbent article is a sanitary napkin or panty liner.

## Patentansprüche

1. Wegwerfbarer absorbierender Artikel zum Tragen anliegend an eine Ausscheidungszone eines Körpers, wobei der genannte Artikel eine dem Körper zugewandte Oberfläche, eine der Kleidung zugewandte Oberfläche und einen absorbierenden Kern zwischen der genannten dem Körper zugewandten Oberfläche und der genannten der Kleidung zugewandten Oberfläche aufweist und weiters eine Längsachse und eine Querachse aufweist, wobei der genannte Artikel eine nachgiebige Gasblase oder eine Vielzahl von nachgiebigen Gasblasen, welche im Verbund wirken, umfaßt, wobei der genannte Artikel dadurch gekennzeichnet ist, daß die Gasblase(n) bei der Herstellung ausschließlich mit Gas bis nicht über 90 % des maximalen Volumens der (jeder) Gasblase gefüllt ist (sind) und eine (Verbund-) Gestalt aufweist (aufweisen), welche eine Ausrichtung aufweist, welche symmetrisch zur Längsachse, aber asymmetrisch zur Querachse des Artikels ist, um die genannte dem Körper zugewandte Oberfläche gegen die Ausscheidungszone für Adaption der genannten dem Körper zugewandten Oberfläche an die Topographie des Trägers in seiner Zone anzuheben.

2. Wegwerfbarer absorbierender Artikel nach Anspruch 1, dadurch gekennzeichnet, daß das genannte Gas Luft ist.

3. Wegwerfbarer absorbierender Artikel nach einem der vorhergehenden Ansprüche, dadurch gekennzeichnet, daß die genannte(n) Gasblase(n) in einer Folie eingeschlossen ist (sind), wobei das Material der genannten Folie vorzugsweise aus der Gruppe aus Polyethylen, Polypropylen, Polyethylenterephthalat, Aluminium, Mischungen derselben oder Laminate von diesen ausgewählt ist.

4. Wegwerfbarer absorbierender Artikel nach einem der vorhergehenden Ansprüche, dadurch gekennzeichnet, daß die genannte Gasblase oder daß die Verbund-Gestalt der Gasblasen entlang der genannten Längsachse länger als entlang der genannten Querachse ist.

5. Wegwerfbarer absorbierender Artikel nach einem der vorhergehenden Ansprüche, dadurch gekennzeichnet, daß die genannte(n) (vereinigten) Gasblase(n) eine Gestalt aufweist (aufweisen), welche aus der Gruppe rechteckiger Gestalten, runder Gestalten, elliptischer Gestalten oder Kombinationen derselben ausgewählt ist.

6. Wegwerfbarer absorbierender Artikel nach einem der vorhergehenden Ansprüche, dadurch gekennzeichnet, daß die genannte dem Körper zugewandte Oberfläche durch ein flüssigkeitsdurchlässiges Substrat beigestellt ist und die genannte der Kleidung zugewandte Oberfläche durch ein flüssigkeitsundurchlässiges Substrat beigestellt ist.

7. Wegwerfbarer absorbierender Artikel nach einem der vorhergehenden Ansprüche, dadurch gekennzeichnet, daß die genannte Gasblase(n) zwischen der genannten der Kleidung zugewandten Oberfläche und dem genannten absorbierenden Kern plaziert ist (sind).

8. Wegwerfbarer absorbierender Artikel nach Anspruch 7, dadurch gekennzeichnet, daß die genannte der Kleidung zugewandte Oberfläche durch eine flüssigkeits- und gasundurchlässige Folie beigestellt ist, wobei jene Folie ebenso einen Teil der genannte(n) Gasblase(n) bildet.

9. Wegwerfbarer absorbierender Artikel nach einem der vorhergehenden Ansprüche, dadurch gekennzeichnet, daß der genannte wegwerfbare absorbierende Artikel eine Hygienevorlage oder eine Slipeinlage ist.

## Revendications

1. Article absorbant jetable destiné à être porté à proximité immédiate d'une zone de décharge corporelle, ledit article comportant une surface faisant face au corps, une surface faisant face au vêtement et une âme absorbante entre ladite surface faisant face au corps et ladite surface faisant face au vêtement et présentant, en outre, un axe longitudinal et un axe latéral, ledit article comprenant une bulle de gaz souple ou un certain nombre de bulles de gaz souples qui fonctionnent de manière combinée, ledit article étant caractérisé en ce que la(les) bulle(s) de gaz est(sont) remplie(s) lors de la fabrication exclusivement avec un gaz ne dépassant pas 90 % du volume maximal de la(chaque) bulle de gaz et a(ont) une forme (combinée) qui a une orientation qui est symétrique par rapport à l'axe longitudinal mais asymétrique par rapport à l'axe latéral de l'article afin de soulever ladite surface faisant face au corps en direction de la zone de décharge en vue de l'adaptation de ladite surface faisant face au corps à la topographie du porteur dans cette zone.

2. Article absorbant jetable selon la revendication 1 caractérisé en ce que ledit gaz est l'air.

3. Article absorbant jetable selon l'une quelconque des revendications précédentes, caractérisé en ce que ladite(lesdites) bulle(s) est(sont) enfermée(s) dans un film, le matériau dudit film étant choisi, de préférence, parmi le groupe constitué de polyéthylène, de polypropylène, de poly(téréphtalate d'éthylène), d'aluminium, de leurs mélanges ou de leurs stratifiés.

4. Article absorbant jetable selon l'une quelconque des revendications précédentes, caractérisé en ce que ladite bulle de gaz ou la forme combinée des bulles de gaz, est plus longue suivant ledit axe longitudinal que suivant ledit axe latéral.

5. Article absorbant jetable selon l'une quelconque des revendications précédentes, caractérisé en ce que ladite(lesdites) bulle(s) de gaz (combinées) a(ont) une forme choisie parmi le groupe constitué de formes rectangulaires, de formes rondes, de formes elliptiques ou de leurs combinaisons.

6. Article absorbant jetable selon l'une quelconque des revendications précédentes, caractérisé en ce que ladite surface faisant face au corps est fournie par un substrat perméable aux liquides, et ladite surface faisant face au vêtement est fournie par un substrat imperméable aux liquides.

7. Article absorbant jetable selon l'une quelconque des revendications précédentes, caractérisé en ce que ladite(lesdites) bulle(s) de gaz est(sont) placée(s) entre ladite surface faisant face au vêtement et ladite âme absorbante.

8. Article absorbant jetable selon la revendication 7, caractérisé en ce que ladite surface faisant face au vêtement est fournie par un film imperméable aux liquides et aux gaz, lequel film fait également partie de ladite(desdites) bulle(s).

9. Article absorbant jetable selon l'une quelconque des revendications précédentes, caractérisé en ce que ledit article absorbant jetable est une serviette hygiénique ou une garniture de culotte.
